# EUROPEAN PATENT SPECIFICATION

(11) **EP 1 383 436 B1**
(45) Date of publication and mention of the grant of the patent: **19.10.2005**
(21) Application number: 01923465.7
(22) Date of filing: 03.05.2001
(51) Int. Cl.: A61B 17/72

(54) **OSTEOSYNTHETIC DEVICE**
OSTEOSYNTHESEVORRICHTUNG
DISPOSITIF OSTEOSYNTHETIQUE

(43) Date of publication of application: 28.01.2004
(73) Proprietor: Synthes AG Chur, 7002 Chur (CH)
(72) Inventor: HEHLI, Markus, CH-7276 Davos Frauenkirch (CH); FERNANDEZ DELL'OCA, Alberto, 11600 Montevideo (UY)
(74) Representative: Lusuardi, Werther Giovanni
(86) International application number: PCT/CH2001/000276
(87) International publication number: WO 2002/089683

(56) References cited:
- EP-A- 0 094 039
- DE-A- 3 835 682
- GB-A- 1 274 470
- US-A- 3 709 218
- US-B1- 6 174 312

## Description

This invention relates to an osteosynthetic device according to the preamble of claim 1, for which the closest prior art is EP-A-0094039.

For the insertion of intramedullary nails the intramedullary canal has to be prepared first. Prior art intramedullary nails have therefore the following major disadvantages:
- the opening is bigger than the nail cross-section because of the bend of the nail; and
- the single bend at one point of the nail does not correspond to the anatomical shape of the medullary cavity of long bones.

Conventional nails forces the surgeon to use in proximal humerus a medial entry point which is located too medially, almost at the articular surface of the humerus, i.e. far from ideal from mechanical and vascular aspects of proximal humerus.

The present invention which is defined in claim 1, is designed to overcome the foregoing problems by providing an osteosynthetic device, particularly an intramedullary nail, which is capable of following the shape of the medullary cavity of long bones of humans. No oversized opening is needed because the helix shape makes it possible to turn the nail during its insertion into the medullary cavity. The entry point of unreamed nails is optimised especially at the femur and the tibia but also in the humerus.

The main advantages of the device according to the invention are the following:
- it allows for a better placement of the entry hole of the nail into the bone avoiding risky points, as for instance, the danger of injuring the vascular supply of the femoral head; this lowers the complication rate and makes humerus nailing easier to perform;
- it does not require an entry hole larger than the cross-section of the nail; and
- it allows easy removal of the nail after bone healing.

Elastic intramedullary nails are not so useful in the adolescent/older child because they may be slightly unstable, requiring often the use of postoperative splints. The use of conventional nails in older children and adolescents is associated to a high risk of femoral head necrosis. A lateral entry points, of a thin constant section (no proximal thick part) can be a high bonus for these patents.

In the case of plates and/or of internal fixators according to the invention the main advantage is the possiblity to allow the implant to be - for instance - anterior in distal humerus and lateral in proximal humerus, avoiding the risk of radial nerve injury.

While one of the principal applications of the invention is as an intramedullary nail, the invention can also be applied to extramedullary devices, e.g. bone plates or internal fixators.

In the modification of a bone nail the device according to the invention may be used in the femur, humerus, tibia and radius.

In a preferred embodiment the envelope of the helix is a circular cylinder having the same central axis as the helix and the helix is running over less than 540°, preferably over less than 360°. The radius r of the circular cylinder purposefully is in the range of 10 to 50 mm, preferably in the range of 15 to 30 mm. The pitch p of the helix should be in the range of 100 to 1'500 mm, preferably in the range of 300 to 1000 mm.
The cross-section orthogonal to the central axis of the helix is preferably a circle, square or star.

In a further preferred embodiment the second end of the nail is pointed, which allows easier introduction into the bone.

In a further preferred embodiment the cross-section orthogonal to the central axis of the helix is essentially a rectangle with the sides a and b, the larger sides b being oriented to the outer and inner side of the helix. Purposefully the ratio of a:b is smaller than 0,50, preferably smaller than 0,35. Preferably the essentially rectangular cross-section is trimmed at its smaller sides a.

In a further preferred embodiment the portion of the helix nearer to the first end is thicker than the portion of the helix nearer to the second end. this allows for attachment of a handle to hold and manipulate the helix nail.

In a further preferred embodiment the central axis of the helix is a straight line.

In a further preferred embodiment the cross-section orthogonal to the central axis has a maximum dimension in the range of 5 to 14 mm and the length of the cylinder or of the helix is in the range of 200 to 500 mm.

In a further preferred embodiment the implant may be provided with lateral holes for locking screws.

The invention will be further described with reference to the accompanying drawings in which:
Fig. 1 is a perspective view of a device according to the invention in the form of a helical nail;
Fig. 2 is a perspective view of a device according to the invention in the form of a helical plate;
Fig. 3 is a detail of the nail according to Fig. 1;
Fig. 4 is a detail of the plate according to Fig. 2;
Fig. 5 is a an orthogonal cross section through the nail according to Fig. 1;
Fig. 6 is a variation of the orthogonal cross section; and
Fig. 7 is a further variation of the orthogonal cross section.

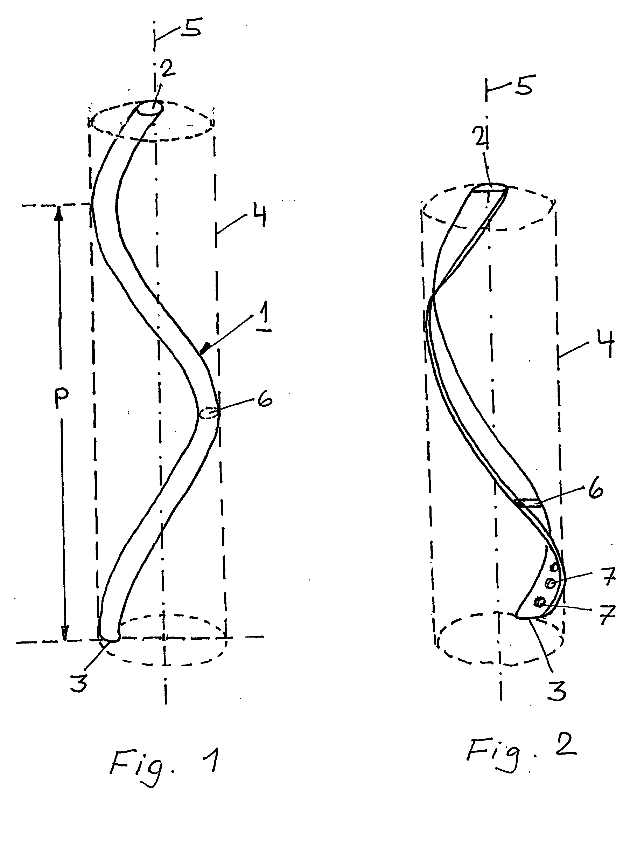

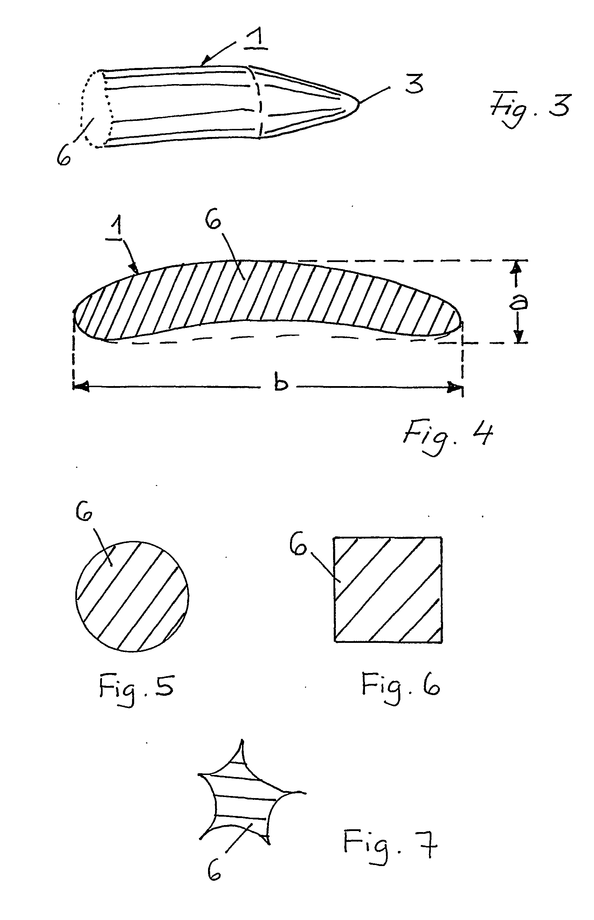

The osteosynthetic device 1 according to the invention is represented in Fig. 1 in the form of an intramedullary nail. It has a longitudinal shape with a central axis 5, a first end 2 an a second end 3. The shape of the device 1 is based on the form of a helix which in geometry is a well-known configuration. The envelope of the helix as shown in Fig. 1 is a circular cylinder 4 having the same central axis 5 as the helix. The central axis 5 of the helix is a straight line. The helix is running over less than 540°, preferably over less than 360°. Typically the helix is running over 240°. The radius r of the circular cylinder 4 is in the range of 10 to 50 mm, preferably in the range of 15 to 30 mm. The pitch p of the helix is range of 100 to 1'500 mm, preferably in the range of 300 to 1000 mm. As shown in Fig. 5 the cross-section orthogonal to the central axis 5 of the helix is a circle, i.e. the helix is made of a cylindrical rod. Alternatively as shown in Figs. 6 and 7 the cross-section may also have the shape of a square or a star (or may be fluted).

Another embodiment of the invention is represented in Fig. 2. It differs from the embodiment of Fig. 1 by the-cross-section orthogonal to the central axis 5 which is not circular but rectangular, i.e. the helix is made of a flattened rod. In particular the cross-section 6 orthogonal to the central axis 5 of the helix is essentially a rectangle with the sides a and b, the larger sides b being oriented to the outer and inner side of the helix. Instead of a rectangular shape the cross-section could have an ellipsoidal shape, where a/2 and b/2 would be the half-axis of the ellipse. The ratio of a:b should be smaller than 0,50, preferably smaller than 0,35.
The portion of the helix nearer to the first end 2 is thicker than the portion of the helix nearer to the second end 3 this allowing attachment of a handle to hold and manipulate the device 1.
The cross-section orthogonal to the central axis 5 has a maximum dimension in the range of 5 to 14 mm.

As shown in Fig. 3 the second end 3 of the device 1 is pointed for easier introduction into the bone.

As shown in Fig. 4 the essentially rectangular cross-section of the device 1 is trimmed at its smaller sides a.

Figs. 5 to 7 show different cross-sections of the nail according to the invention.

The devices according to the invention may be made of any appropriate material, depending on the purpose to be served. They may be made of metals, for example an appropriate stainless steel, titanium or polymeric material in particular of composite nature.

## Claims

1. An osteosynthetic device (1), in particular an intramedullary nail, having a longitudinal shape with a central axis (5), a first end (2) and a second end (3), the shape of the device (1) being based on the form of a helix,
**characterized in that**
a) the cross-section orthogonal to the central axis (5) has a maximum dimension in the range of 5 to 14 mm;
b) the helix is running over less than 540°; and
c) the pitch p of the helix is in the range of 100 to 1'500 mm.

2. Device (1) according to claim 1, **characterized in that** the helix is running over less than 360°.

3. Device (1) according to claim 1 or 2, **characterized in that** the cross-section orthogonal to the central axis (5) has a maximum dimension in the range of 7 to 11 mm.

4. Device (1) according to one of the claims 1 to 3, **characterized in that** the pitch p of the helix is in the range of 300 to 1000 mm.

5. Device (1) according to one of the claims 1 to 4, **characterized in that** the pitch p of the helix is larger than 400 mm, preferably larger than 600 mm.

6. Device (1) according to one of the claims 1 to 5, **characterized in that** the envelope of the helix is a circular cylinder (4) having the same central axis (5) as the helix.

7. Device (1) according to one of the claims 1 to 6, **characterized in that** the radius r of the circular cylinder (4) is in the range of 10 to 50 mm, preferably in the range of 15 to 30 mm.

8. Device (1) according to one of the claims 1 to 7, **characterized in that** a cross-section (6) orthogonal to the central axis (5) of the helix is a circle.

9. Device (1) according to one of the claims 1 to 7, **characterized in that** a cross-section (6) orthogonal to the central axis (5) of the helix is a square or star.

10. Device (1) according to one of the claims 1 to 9, **characterized in that** the second end (3) is pointed.

11. Device (1) according to one of the claims 1 to 10, **characterized in that** a cross-section (6) orthogonal to the central axis (5) of the helix is essentially a rectangle with the sides a and b, the larger sides b being oriented to the outer and inner side of the helix.

12. Device (1) according to claim 11, **characterized in that** the ratio of a:b is smaller than 0,50, preferably smaller than 0,35.

13. Device (1) according to claim 11 or 12, **characterized in that** the essentially rectangular cross-section is trimmed at its smaller sides a.

14. Device (1) according to one of the claims 1 to 13, **characterized in that** the portion of the helix nearer to the first end (2) is thicker than the portion of the helix nearer to the second end (3).

15. Device (1) according to one of the claims 1 to 14, **characterized in that** the central axis (5) of the helix is a straight line.

16. Device (1) according to one of the claims 1 to 15, **characterized in that** the length of the cylinder or of the helix is in the range of 200 to 500 mm, preferably in the range of 250 to 400 mm.

17. Device (1) according to one of the claims 1 to 16, **characterized in that** it is provided with through-going holes (7) for locking screws, preferably near the second end (3).

18. Device (1) according to one of the claims 1 to 17, **characterized in that** it is provided with at least two, preferably with at least three trough-going holes (7) for locking screws.

## Patentansprüche

1. Osteosynthesevorrichtung (1), insbesondere Marknagel, welcher eine langgestreckte Form mit einer Zentralachse (5), einem ersten Ende (2) und einem zweiten Ende (3) aufweist, wobei die Form der Vorrichtung (1) auf der Form einer Helix basiert, **dadurch gekennzeichnet, dass**
a) der orthogonal zu der Zentralachse (5) verlaufende Querschnitt eine Maximalabmessung in dem Bereich von 5 bis 14 mm aufweist;
b) die Helix sich über weniger als 540 ° erstreckt; und
c) die Steigung p der Helix in dem Bereich von 100 bis 1500 mm liegt.

2. Vorrichtung (1) nach Anspruch 1, **dadurch gekennzeichnet, dass** die Helix sich über weniger als 360 ° erstreckt.

3. Vorrichtung (1) nach Anspruch 1 oder 2, **dadurch gekennzeichnet, dass** der orthogonal zu der Zentralachse (5) verlaufende Querschnitt eine Maximalabmessung in dem Bereich von 7 bis 11 mm aufweist.

4. Vorrichtung (1) nach einem der Ansprüche 1 bis 3, **dadurch gekennzeichnet, dass** die Steigung p der Helix in dem Bereich von 300 bis 1000 mm liegt.

5. Vorrichtung (1) nach einem der Ansprüche 1 bis 4, **dadurch gekennzeichnet, dass** die Steigung p der Helix grösser als 400 mm, vorzugsweise grösser als 600 mm ist.

6. Vorrichtung (1) nach einem der Ansprüche 1 bis 5, **dadurch gekennzeichnet, dass** die Hüllkurve der Helix ein Kreiszylinder (4) ist, welcher dieselbe Zentralachse (5) wie die Helix aufweist.

7. Vorrichtung (1) nach einem der Ansprüche 1 bis 6, **dadurch gekennzeichnet, dass** der Radius r des Kreiszylinders (4) in dem Bereich von 10 bis 50 mm, vorzugsweise in dem Bereich von 15 bis 30 mm liegt.

8. Vorrichtung (1) nach einem der Ansprüche 1 bis 7, **dadurch gekennzeichnet, dass** ein orthogonal zu der Zentralachse (5) der Helix verlaufender Querschnitt (6) einen Kreis darstellt.

9. Vorrichtung (1) nach einem der Ansprüche 1 bis 7, **dadurch gekennzeichnet, dass** ein orthogonal zu der Zentralachse (5) der Helix verlaufender Querschnitt (6) ein Rechteck oder einen Stern darstellt.

10. Vorrichtung (1) nach einem der Ansprüche 1 bis 9, **dadurch gekennzeichnet, dass** das zweite Ende (3) zugespitzt ist.

11. Vorrichtung (1) nach einem der Ansprüche 1 bis 10, **dadurch gekennzeichnet, dass** ein orthogonal zu der Zentralachse (5) der Helix verlaufender Querschnitt (6) im wesentlichen ein Rechteck mit den Seiten a und b darstellt, wobei die grösseren Seiten b jeweils zu der Aussenseite bzw. der Innenseite der Helix hin ausgerichtet sind.

12. Vorrichtung (1) nach Anspruch 11, **dadurch gekennzeichnet, dass** das Verhältnis von a:b kleiner als 0,50, vorzugsweise kleiner als 0,35 ist.

13. Vorrichtung (1) nach Anspruch 11 oder 12, **dadurch gekennzeichnet, dass** der im wesentlichen rechteckige Querschnitt an seinen kleineren Seiten a beschnitten ist.

14. Vorrichtung (1) nach einem der Ansprüche 1 bis 13, **dadurch gekennzeichnet, dass** der näher an dem ersten Ende (2) gelegene Abschnitt der Helix dicker ist als der näher an dem zweiten Ende (3) gelegene Abschnitt der Helix.

15. Vorrichtung (1) nach einem der Ansprüche 1 bis 14, **dadurch gekennzeichnet, dass** die Zentralachse (5) der Helix eine gerade Linie ist.

16. Vorrichtung (1) nach einem der Ansprüche 1 bis 15, **dadurch gekennzeichnet, dass** die Länge des Zylinders bzw. der Helix in dem Bereich von 200 bis 500 mm, vorzugsweise in dem Bereich von 250 bis 400 mm liegt.

17. Vorrichtung (1) nach einem der Ansprüche 1 bis 16, **dadurch gekennzeichnet, dass** sie mit vorzugsweise in der Nähe des zweiten Endes (3) angeordneten, durchgehenden Löchern (7) für Verriegelungsschrauben versehen ist.

18. Vorrichtung (1) nach einem der Ansprüche 1 bis 17, **dadurch gekennzeichnet, dass** sie mit zumindest zwei, vorzugsweise mit zumindest drei durchgehenden Löchern (7) für Verriegelungsschrauben versehen ist.

## Revendications

1. Dispositif d'ostéosynthèse (1), notamment clou intramédullaire présentant une forme allongée avec un axe central (5), une première extrémité (2) et une deuxième extrémité (3), la forme du dispositif (1) étant basée sur la forme d'une hélice, **caractérisé en ce que**
a) la section transversale s'étendant orthogonalement à l'axe central (5) présente une dimension maximale comprise entre 5 et 14 mm;
b) l'hélice s'étend sur moins de 540°; et
c) le pas p de l'hélice est compris entre 100 et 1500 mm.

2. Dispositif (1) selon la revendication 1, **caractérisé en ce que** l'hélice s'étend sur moins de 360°.

3. Dispositif (1) selon la revendication 1 ou 2, **caractérisé en ce que** la section transversale s'étendant orthogonalement à l'axe central (5) présente une dimension maximale comprise entre 7 et 11 mm.

4. Dispositif (1) selon l'une des revendications 1 à 3, **caractérisé en ce que** le pas p de l'hélice est compris entre 300 et 1 000 mm.

5. Dispositif (1) selon l'une des revendications 1 à 4, **caractérisé en ce que** le pas p de l'hélice est supérieur à 400 mm, de préférence supérieur à 600 mm.

6. Dispositif (1) selon l'une des revendications 1 à 5, **caractérisé en ce que** l'enveloppe de l'hélice est un cylindre circulaire (4) présentant le même axe central (5) que l'hélice.

7. Dispositif (1) selon l'une des revendications 1 à 6, **caractérisé en ce que** le rayon r du cylindre circulaire (4) est compris entre 10 et 50 mm, de préférence entre 15 et 30 mm.

8. Dispositif (1) selon l'une des revendications 1 à 7, **caractérisé en ce qu'**une section transversale (6) s'étendant orthogonalement à l'axe central (5) est un cercle.

9. Dispositif (1) selon l'une des revendications 1 à 7, **caractérisé en ce qu'**une section transversale (6) s'étendant orthogonalement à l'axe central (5) de l'hélice est un rectangle ou une étoile.

10. Dispositif (1) selon l'une des revendications 1 à 9, **caractérisé en ce que** la deuxième extrémité (3) est pointue.

11. Dispositif (1) selon l'une des revendications 1 à 10, **caractérisé en ce que** la section transversale (6) s'étendant orthogonalement à l'axe central (5) de l'hélice est essentiellement un rectangle présentant les côtés a et b, les grands côtés b étant respectivement orientés vers le côté extérieur et le côté intérieur de l'hélice.

12. Dispositif (1) selon la revendication 11, **caractérisé en ce que** le rapport a:b est inférieur à 0,50, de préférence inférieur à 0,35.

13. Dispositif (1) selon la revendication 11 ou 12, **caractérisé en ce que** la section essentiellement rectangulaire est coupée au niveau de ses petits côtés a.

14. Dispositif (1) selon l'une des revendications 1 à 13, **caractérisé en ce que** la partie de l'hélice située plus près de la première extrémité (2) est plus épaisse que la partie de l'hélice située plus près de la deuxième extrémité (3).

15. Dispositif (1) selon l'une des revendications 1 à 14, **caractérisé en ce que** l'axe central (5) de l'hélice est une ligne droite.

16. Dispositif (1) selon l'une des revendications 1 à 15, **caractérisé en ce que** la longueur du cylindre ou de l'hélice est comprise entre 200 et 500 mm, de préférence entre 250 et 400 mm.

17. Dispositif (1) selon l'une des revendications 1 à 16, **caractérisé en ce qu'**il est pourvu de trous traversants (7) pour vis de verrouillage disposés de préférence à proximité de la deuxième extrémité (3).

18. Dispositif (1) selon l'une des revendications 1 à 17, **caractérisé en ce qu'**il est pourvu d'au moins deux, de préférence d'au moins trois trous traversants (7) pour vis de verrouillage.
